# EUROPEAN PATENT APPLICATION

(11) **EP 3 459 927 A1**
(43) Date of publication of application: **27.03.2019**
(21) Application number: 17799184.1
(22) Date of filing: 01.05.2017
(51) Int. Cl.: C07C 67/08, C07C 67/14, C07C 67/52, C07C 69/75

(54) **COMPOUND MANUFACTURING METHOD, COMPOUND, AND MIXTURE**

(30) Priority: 18.05.2016 JP 2016100012
(71) Applicant: Zeon Corporation, Tokyo 100-8246 (JP)
(72) Inventor: SAKAMOTO, Kei, Tokyo 100-8246 (JP); OKUYAMA, Kumi, Tokyo 100-8246 (JP)
(74) Representative: Beckmann, Claus
(86) International application number: PCT/JP2017/017182
(87) International publication number: WO 2017/199754

(57) **Abstract**

Disclosed is a method of producing a compound which includes: obtaining a reaction product which contains a monoester compound represented by the following formula (I) and 1,4-cyclohexanedicarboxylic acid dichloride and/or 1,4-cyclohexanedicarboxylic acid, by reacting in an organic solvent a hydroxy compound represented by the formula Q-OH (where Q represents organic group which may have a substituent) with 1,4-cyclohexanedicarboxylic acid dichloride and/or 1,4-cyclohexanedicarboxylic acid; and mixing the reaction product with an acidic aqueous solution, 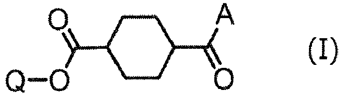 where A represents hydroxyl group or chlorine atom and Q is as defined above.

## Description

### TECHNICAL FIELD

The present disclosure relates to methods of producing compounds which can be intermediates of polymerizable compounds usable for the manufacture of optical films and optically anisotropic products; compounds; and mixtures containing the compounds.

### BACKGROUND

Phase difference plates used in flat panel display devices and other like devices include quarter-wave plates that convert linearly polarized light into circularly polarized light, and half-wave plates that rotate the plane of vibration of linearly polarized light by 90 degrees. These phase difference plates can achieve exact λ/4 or λ/2 phase difference for particular monochromatic light.

However, the conventional phase difference plates have the drawback of undesirably converting the polarized light emitting from the phase difference plate into colored one. The cause of this is that the material of the phase difference plate has wavelength dispersion of phase difference and white light, or composite waves which include different rays in the visible range, shows a distribution of polarization states at different wavelengths and hence incident light cannot be converted into polarized light having its phase retarded by exactly λ/4 or λ/2 over the entire wavelength range.

To address such a drawback, studies have been made for wide-band phase difference plates which may provide uniform phase difference over a wide wavelength range, i.e., phase difference plates having reversed wavelength dispersion.

On the other hand, improvements in the function of portable information terminals such as mobile PCs and cellular phones and their widespread use are increasingly requiring that flat panel display devices be thinned as much as possible. Correspondingly, it is also required to make thinner the phase difference plates which constitute the flat panel display devices.

The method of making thinner phase difference plates which is deemed most effective in recent years involves applying polymerizable compositions containing low-molecular weight polymerizable compounds on film substrates to form optical films. This led to many developments of polymerizable compounds or polymerizable compositions containing the polymerizable compounds that allow for the manufacture of optical films that have superior reverse wavelength dispersion.

As preferred compounds which can be intermediates of such polymerizable compounds, carboxylic acid ester compounds having one or more carboxyl groups are suitably used. Various production methods have been studied to produce carboxylic acid ester compounds having one or more carboxyl groups.

One of the production methods which have heretofore been studied involves reacting alcohols with dicarboxylic acid chlorides (see, e.g., PTL 1). Another production method includes mixing a compound (A) having one hydroxyl group with a compound (B) having two or more carboxyl groups to cause to esterification (step 1); mixing the reaction mixture obtained from step 1 with a basic compound and water to afford a suspension (step 2); and obtaining, from the suspension obtained in step 2, solids containing a carboxylic acid ester compound having one or more carboxyl groups (step 3) (see, e.g., PTL 2).

### CITATION LIST

### Patent Literature

[PTL 1] JPS62289545A
[PTL 2] JP2015157776A

### SUMMARY

### (Technical Problem)

Industrial-scale manufacture of optical films or optically anisotropic products (hereinafter also collectively referred to as "optical films etc.") requires efficient production of polymerizable compounds. To that end, there was a need in the art to achieve high-efficient production of a high-quality compound which can be an intermediate of a polymerizable compound (hereinafter also referred to as an "intermediate compound").

However, the production methods described in PTL 1 and PTL 2 were not able to achieve both high production efficiency and high product quality at the same time when producing such an intermediate compound.

The present disclosure was made in view of the circumstances described above and an object of the present disclosure is to provide a method of producing a compound, which is capable of high-efficient production of a high-quality intermediate compound.

Another object of the present disclosure is to provide a compound which can be a precursor of the high-quality intermediate compound, and a mixture containing the compound.

### (Solution to Problem)

The inventors conducted considerable studies to solve the foregoing problem and discovered that high-efficient production of a high-quality intermediate compound is possible by esterifying a hydroxy compound having a specific structure with a specific carboxylic acid compound in organic solvent to afford a reaction product and mixing the reaction product with at least one liquid having a specific property. The inventors thus completed the present disclosure.

Therefore, according to the present disclosure, there are provided a method of producing a compound, a compound and a mixture, which are described below.
[1] A method of producing a compound, comprising:
   a step (A1) of obtaining a reaction product which contains a monoester compound represented by the following formula (I) and 1,4-cyclohexanedicarboxylic acid dichloride and/or 1,4-cyclohexanedicarboxylic acid, by reacting in an organic solvent a hydroxy compound represented by the formula Q-OH (where Q represents organic group which may have a substituent) with 1,4-cyclohexanedicarboxylic acid dichloride and/or 1,4-cyclohexanedicarboxylic acid; and
   a step (A2) of mixing the reaction product with an acidic buffer solution, 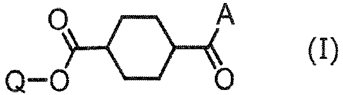
   where A represents hydroxyl group or chlorine atom and Q is as defined above.
[2] The method according to [1], wherein the organic solvent is a hydrophilic organic solvent.
[3] The method according to [1] or [2], further comprising a step (A1-1) of mixing the reaction product with water between the step (A1) and the step (A2).
[4] The method according to any one of [1] to [3], wherein the acidic buffer solution has a pH of 5.0 or more and 6.0 or less.
[5] A method of producing a compound, comprising:
   a step (B1) of obtaining a reaction solution which contains a monoester compound represented by the following formula (I) and 1,4-cyclohexanedicarboxylic acid dichloride and/or 1,4-cyclohexanedicarboxylic acid, by reacting in an organic solvent a hydroxy compound represented by the formula Q-OH (where Q represents organic group which may have a substituent) with 1,4-cyclohexanedicarboxylic acid dichloride or 1,4-cyclohexanedicarboxylic acid;
   a step (B2) of mixing the reaction solution with a basic compound and water to afford a precipitate; and
   a step (B3) of mixing the precipitate obtained in step (B2), either in solid or dissolved state, with an acidic aqueous solution, 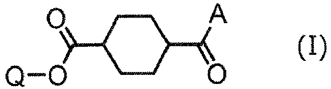
   where A represents hydroxyl group or chlorine atom and Q is as defined above.
[6] The method according to [5], wherein the acidic aqueous solution has an acid dissociation constant pKa of 6.5 or less.
[7] The method according to [5], wherein the acidic aqueous solution is an acidic buffer solution having a pH of 5.0 or more and 6.0 or less.
[8] The method according to any one of [1] to [4] and [7], wherein the acidic buffer solution is a mixed solution of acetic acid and sodium acetate, or a mixed solution of potassium hydrogen phthalate and sodium hydroxide.
[9] The method according to any one of [1] to [8], wherein the organic group Q of the compound represented by the formula (I) is represented by the following formula (II): 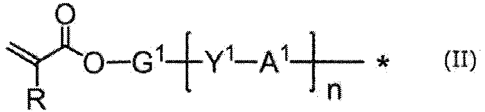 where R represents hydrogen or methyl group; Y¹ represents chemical single bond, -O-, -S-, -O-C(=O)-, -C(=O)-O-, -O-C(=O)-O-, -NR¹-C(=O)-, or -C(=O)-NR¹- (where R¹ represents hydrogen atom or C1-C6 alkyl group); G¹ represents C1-C20 divalent chain aliphatic group which may have a substituent (where the chain aliphatic group may include -O-, -S-, -O-C(=O)-, -C(=O)-O-, -O-C(=O)-O-, -NR²-C(=O)-, -C(=O)-NR²--NR²-, or -C(=O)- (where R² represents hydrogen atom or C1-C6 alkyl group) as a spacer, with the proviso that two or more of -O- or two or more of -S- are not present adjacent to each other); A¹ represents C3-C12 divalent aromatic hydrocarbon ring which may have a substituent or C3-C12 divalent cycloaliphatic group which may have a substituent; and n represents 0 or 1.
[10] A compound represented by the following formula (III): 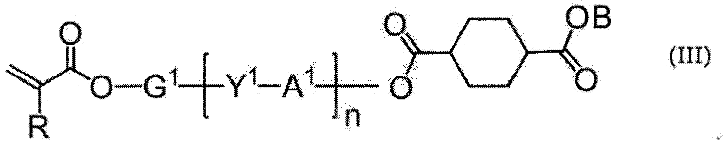 where R represents hydrogen or methyl group; Y¹ represents chemical single bond, -O-, -S-, -O-C(=O)-, -C(=O)-O-, -O-C(=O)-O-, -NR¹-C(=O)-, or -C(=O)-NR¹- (where R¹ represents hydrogen atom or C1-C6 alkyl group); G¹ represents C1-C20 divalent chain aliphatic group which may have a substituent (where the chain aliphatic group may include -O-, -S-, -O-C(=O)-, -C(=O)-O-, -O-C(=O)-O-, -NR²-C(=O)-, -C(=O)-NR²-, -NR²-, or -C(=O)- (where R² represents hydrogen atom or C1-C6 alkyl group) as a spacer, with the proviso that two or more of -O- or two or more of -S- are not present adjacent to each other); A¹ represents C3-C12 divalent aromatic hydrocarbon ring which may have a substituent or C3-C12 divalent cycloaliphatic group which may have a substituent; B represents alkali metal atom or alkaline earth metal atom; and n represents 0 or 1.
[11] A mixture comprising:
   the compound represented by the formula (III) defined in [10]; and
   a compound represented by the following formula (IV),
   wherein the mixture contains the compound (IV) in an amount of 50 mol% or less based on a total amount of the compound (111) and the compound (IV) and contains 1,4-cyclohexanedicarboxylic acid and/or 1,4-cyclohexanedicarboxylic acid dichloride in an amount of less than 5 mol% of the total mixture, 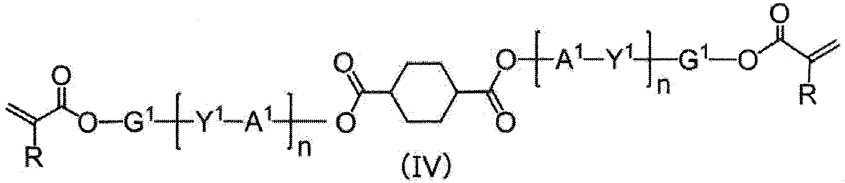
   where R, Y¹, G¹ and A¹ each represent the same group or bond as defined in the formula (III); and n has the same value as defined in the formula (III).
[12] The compound according to [10], wherein in the formula (III), G¹ is unsubstituted hexyl group, Y¹ is -O-, A¹ is unsubstituted 1,4-phenylene group, and n is 1.
[13] The mixture according to [11], wherein in the formulas (III) and (IV), G¹ is unsubstituted hexyl group, Y¹ is -O-, A¹ is unsubstituted 1,4-phenylene group, and n is 1.

### (Advantageous Effect)

According to the present disclosure, it is possible to provide a production method capable of producing a high-quality intermediate compound at high efficiency.

According to the present disclosure, it is also possible to provide a compound which can be a precursor of the high-quality intermediate compound, and a mixture containing the compound.

### BRIEF DESCRIPTION OF THE DRAWINGS

In the accompanying drawings:
FIG. 1 is an infrared absorption spectrum of a mixture containing an intermediate compound obtained by a production method according to an example of the present disclosure; and
FIG. 2 is an infrared absorption spectrum of a white solid containing a compound according to an example of the present disclosure, which can be a precursor of an intermediate compound.

### DETAILED DESCRIPTION

The present disclosure will be described in detail below. As used herein, the phrase "may have a substituent" means "substituted or unsubstituted." Further, it is defined herein that when an organic group such as an alkyl group or an aromatic hydrocarbon ring group in a general formula has a substituent, the number of carbon atoms of the organic group having a substituent excludes the number of carbon atoms of the substituent. For example, when a C3-C12 aromatic hydrocarbon ring group has a substituent, the number of carbon atoms of the C3-C12 aromatic hydrocarbon ring group excludes the number of carbon atoms of such a substituent.

The production method according to the present disclosure can be used in any application, e.g., for the production of an intermediate compound of a polymerizable compound usable for the manufacture of an optical film or an optically anisotropic product. The compound of the present disclosure and a mixture containing the compound can be used in any application, e.g., for the production of an intermediate compound of a polymerizable compound used for the manufacture of optical films and optically anisotropic products. The compound of the present disclosure and a mixture containing the compound can be produced by any method, e.g., the production method of the present disclosure.

### (Method of Producing Compound)

Hereinafter, two general methods A and B will be described for the production of the compound of the present disclosure. Production methods A and B are common in that they start with a step of esterifying a hydroxy compound having a specific structure with 1,4-cyclohexanedicarboxylic acid dichloride and/or 1,4-cyclohexanedicarboxylic acid to afford a reaction product containing a monoester compound having a specific structure (i.e., step (A1) or step (B1) described below in detail), but differ in treatment steps until an intermediate compound of desired quality is obtained from the reaction product. Each method will be described below.

### <Production Method A>

Production method A includes a step (A1) of obtaining a reaction product which contains a monoester compound having the following formula (I) and 1,4-cyclohexanedicarboxylic acid dichloride and/or 1,4-cyclohexanedicarboxylic acid, the reaction product obtained by reacting in an organic solvent a hydroxy compound represented by the formula Q-OH (where Q represents organic group which may have a substituent) with 1,4-cyclohexanedicarboxylic acid dichloride and/or 1,4-cyclohexanedicarboxylic acid; and a step (A2) of mixing the reaction product with an acidic buffer solution. 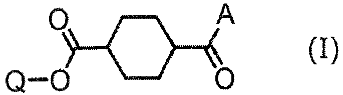 where A represents hydroxyl group or chlorine atom and Q represents organic group which may have a substituent.

Through these steps, it is possible to obtain a mixture containing an intermediate compound of interest represented by the formula (I) where A is hydroxyl group.

### -Step (A1)-

In step (A1), 1,4-cyclohexanedicarboxylic acid and/or 1,4-cyclohexanedicarboxylic acid dichloride are/is reacted with a hydroxy compound having a specific structure in an organic solvent optionally in the presence of a base to esterify the hydroxy compound having a specific structure.

### [Organic Solvent]

Any organic solvent can be used and those known in the art can be used. In particular, it is preferred to use a hydrophilic organic solvent. The term "hydrophilic organic solvent" as used herein refers to an organic solvent having a solubility in 100 g of water at 25°C of greater than 10 (g/100g-H₂O). Specific examples of hydrophilic organic solvents include aprotic polar solvents such as N-methyl-2-pyrrolidone, N,N-dimethylacetamide, N,N-imidazolidinone, and γ-butyrolactone; and ether solvents such as tetrahydrofuran and dioxane. Two or more of these hydrophilic organic solvents may be mixed. Preferred are N-methyl-2-pyrrolidone (NMP) and tetrahydrofuran (THF).

### [Hydroxy Compound]

As the hydroxy compound represented by the formula Q-OH (where Q represents organic group which may have a substituent), a hydroxy compound whose organic group Q is represented by the following formula (II) is preferred: 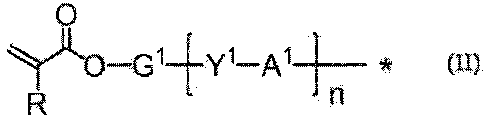 where R represents hydrogen atom or methyl group; Y¹ represents chemical single bond, -O-, -S-, -O-C(=O)-, -C(=O)-O-, -O-C(=O)-O-, -NR¹-C(=O)-, or -C(=O)-NR¹- (where R¹ represents hydrogen atom or C1-C6 alkyl group); G¹ represents C1-C20 divalent chain aliphatic group which may have a substituent (where the chain aliphatic group may include -O-, -S-, -O-C(=O)-, -C(=O)-O-, -O-C(=O)-O-, -NR²-C(=O)-, -C(=O)-NR²-, -NR²-, or -C(=O)- (where R² represents hydrogen atom or C1-C6 alkyl group) as a spacer, with the proviso that two or more of -O- or two or more of -S- are not present adjacent to each other); A¹ represents C3-C12 divalent aromatic hydrocarbon ring which may have a substituent or C3-C12 divalent cycloaliphatic group which may have a substituent; and n represents 0 or 1.

### [1,4-Cyclohexanedicarboxylic Acid and 1,4-Cyclohexanedicarboxylic Acid Dichloride]

As the 1,4-cyclohexanedicarboxylic acid used in step (A1), trans-1,4-cyclohexanedicarboxylic acid is preferred, and as the 1,4-cyclohexanedicarboxylic acid dichloride, trans-1,4-cyclohexanedicarboxylic acid dichloride is preferred. The 1,4-cyclohexanedicarboxylic acid and 1,4-cyclohexanedicarboxylic acid dichloride can be used singly or as a mixture of both, but it is preferred to use either of them singly.

### [Base]

In step (A1), a base can optionally be blended in the organic solvent. Examples of bases include triethylamine, diisopropylethylamine, and 4-(dimethylamino)pyridine. The esterification reaction in the presence of a base can improve the esterification efficiency of the hydroxy compound having the specific structure.

### [Condensing Agent]

In step (A1), a condensing agent can be optionally used. Examples of condensing agents include dicyclohexylcarbodiimide, diisopropylcarbodiimide, 1-ethyl-3-(3-dimethylaminopropyl)carbodiimide, 1-(3-dimethylaminopropyl)-3-ethylcarbodiimide hydrochloride, and bis(2,6-diisopropylphenyl)carbodiimide, with 1-(3-dimethylaminopropyl)-3-ethylcarbodiimide hydrochloride being preferred.

### [Esterification Reaction]

By the esterification reaction in step (A1), a diester compound represented by the following formula (V) is produced in addition to the monoester compound represented by the formula (1) shown above: 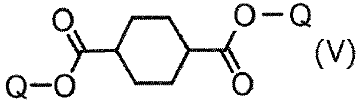 where Q represents organic group which may have a substituent, preferably organic group represented by the formula (II) shown above.

Further, unreacted 1,4-cyclohexanedicarboxylic acid and/or 1,4-cyclohexanedicarboxylic acid dichloride may remain in the reaction product.

When reacting 1,4-cyclohexanedicarboxylic acid or 1,4-cyclohexanedicarboxylic acid dichloride alone with the hydroxy compound having the specific structure in the esterification reaction, it is preferred to use 1,4-cyclohexanedicarboxylic acid or 1,4-cyclohexanedicarboxylic acid dichloride in an amount of 0.8 moles or more and 10 moles or less per mole of the hydroxy compound. When reacting a mixture of 1,4-cyclohexanedicarboxylic acid and 1,4-cyclohexanedicarboxylic acid dichloride with the hydroxy compound, it is preferred that the ratio of the total amount of the mixture of 1,4-cyclohexanedicarboxylic acid and 1,4-cyclohexanedicarboxylic acid dichloride to the amount of the hydroxy compound used falls within the range described above. Further, it is preferred that the total amount of moles of 1,4-cyclohexanedicarboxylic acid and 1,4-cyclohexanedicarboxylic acid dichloride is 0.8 moles or more and 10 moles or less per mole of the hydroxy compound.

When the base described above is used in the esterification reaction, it is preferred to use the base in an amount of 0.05 moles or more and 5 moles or less per mole of the hydroxy compound. When the condensing agent described above is used in the esterification reaction, it is preferred to use the condensing agent in an amount of 0.5 moles or more and 10 moles or less per mole of the hydroxy compound.

The temperature during the esterification reaction is preferably -20°C or higher and 120°C or lower. From the viewpoint of the esterification efficiency, suitable temperature conditions differ depending on the raw materials to be used, i.e., whether 1,4-cyclohexanedicarboxylic acid and/or 1,4-cyclohexanedicarboxylic dichloride are/is used. For example, when 1,4-cyclohexanedicarboxylic acid is used, it is more preferred to set the reaction temperature to -10°C or higher and 80°C or lower. When 1,4-cyclohexanedicarbon dichloride is used, it is more preferred to set the reaction temperature to 40°C or lower.

When a mixture of 1,4-cyclohexanedicarboxylic acid and 1,4-cyclohexanedicarboxylic dichloride is used, the reaction temperature is the same as that when 1,4-cyclohexanedicarboxylic acid is used.

The esterification reaction time preferably ranges from 1 minute to 72 hours, more preferably from 1 to 48 hours, even more preferably from 1 to 24 hours.

### -Step (A2)-

In step (A2), the reaction product obtained in step (A1) is mixed with an acidic buffer solution. This step allows, on one hand, 1,4-cyclohexanedicarboxylic acid dichloride and/or 1,4-cyclohexanedicarboxylic acid-unreacted matter contained in the reaction product obtained in step (A1)-to be dissolved in an aqueous phase derived from the acidic buffer solution and, on the other hand, allows the monoester compound represented by the formula (I) where A is hydroxyl group to be precipitated. Of note, when 1,4-cyclohexanedicarboxylic acid dichloride was used in step (A1), the reaction product obtained in step (A1) contains a monoester compound represented by the formula (I) where A is chlorine atom. The monoester compound represented by the formula (I) where A is chlorine atom has the moiety -C(=O)-Cl. When a reaction product containing a monoester compound having such a structure is mixed with an acidic buffer solution in step (A2), the moiety -C(=O)-Cl is hydrolyzed into carboxyl group. As a result, in step (A2), a monoester compound represented by the formula (I) where A is hydroxyl group, i.e., an intermediate compound of interest can be precipitated.

### [Acidic Buffer Solution]

The acidic buffer solution preferably has a pH of 5.0 or more and 6.0 or less because 1,4-cyclohexanedicarboxylic acid and/or 1,4-cyclohexanedicarboxylic acid dichloride remaining in the reaction product can well dissolve therein. Any acidic buffer solution can be used as long as the pH falls within the above range. Preferred is a mixed solution of acetic acid and sodium acetate or a mixed solution of potassium hydrogen phthalate and sodium hydroxide, with a mixed solution of acetic acid and sodium acetate being more preferred. Any blending ratio of the compounds can be used so long as the pH of the mixed solution falls within the referred range described above.

The term "buffer solution" as used herein means a solution having a buffering capacity.

The "pH" of the acidic buffer solution herein can be measured by the method described in Examples.

### [Mixing]

Any amount of the acidic buffer solution can be used in step (A2) and the amount can be 100 parts by mass or more and 5,000 parts by mass or less per 100 parts by mass of the hydroxy compound used in step (A1).

The mixing time in step (A2) preferably ranges from 1 minute to 24 hours, more preferably 1 minute to 12 hours, even more preferably 1 minute to 3 hours.

### -Step (Al-1)-

Optionally, a step (A1-1) of mixing the reaction product obtained in step (A1) with water can be carried out between steps (A1) and (A2). With this step, when A is chlorine atom in the general formula (I) shown above, the moiety -C(=O)-Cl of the acid chloride can be hydrolyzed into carboxyl group. Similarly, the moiety -C(=O)-Cl of the residual unreacted 1,4-cyclohexanedicarboxylic acid dichloride can be hydrolyzed into carboxyl group. As a consequence, it is possible to reduce the amount of the acidic buffer solution used in step (A2) to thereby enhance the production efficiency of the intermediate compound.

### [Water]

Any type of water can be added in step (A1-1), e.g., distilled water or ion-exchanged water can be used.

### [Mixing]

Any amount of water can be used in step (A1-1) and the amount can be 10 parts by mass or more and 5,000 parts by mass or less per 100 parts by mass of the hydroxy compound used in step (A1).

The mixing time in step (A1-1) preferably ranges from 1 minute to 24 hours, more preferably 1 to 12 hours, even more preferably 1 to 6 hours.

### [Solid-Liquid Separation and Washing]

In step (A1-1), after mixing the reaction product with water, solids contained in the mixed solution may be recovered and washed. Any method can be used to recover solids and examples include filtration and decantation. Preferably, solids are recovered by filtration. The recovered solids may be washed with any medium, e.g., buffer solution, water, or water-methanol solvent. Washing is preferably carried out using water or water-methanol solvent. When using water-methanol solvent, it is particularly preferred to use a 1:1 (by mass) water-methanol solvent. When a buffer solution is used for washing in this step, any buffer solution can be used, e.g., common buffer solutions or acidic buffer solutions mentioned in step (A2) above can be used. The term "washing" as used herein means an operation of contacting a solid with a solvent. The washed solid can be dried, for example, by general drying means such as a vacuum dryer.

When step (A2) is carried out after step (A1-1), optionally, the solids obtained by the washing can be in dissolved state by being dissolved in any desired solvent before step (A2). Any solvent can be used to dissolve the solids, e.g., common organic solvents or hydrophilic organic solvents described above can be used. In particular, it is preferred to use hydrophilic organic solvents. The organic solvent used in step (A1) and the organic solvent used for dissolution of solids may be the same or different.

### -Solid/Liquid Separation Step-

A slurry containing the monoester compound represented by the formula (I) precipitated in step (A2) is subjected to solid separation for example by filtration or decantation for recovery of solids. Preferably, filtration is used to recover a mixture containing an intermediate compound of interest. The solids obtained can then be washed and dried by common methods similar to those described in the washing operation in step (A1-1) to afford an intermediate compound of interest and a mixture which contains the intermediate compound.

### <Production Method B>

Production method B includes a step (B1) of obtaining a reaction solution which contains a monoester compound having the following formula (1) and 1,4-cyclohexanedicarboxylic acid dichloride and/or 1,4-cyclohexanedicarboxylic acid, by reacting in an organic solvent a hydroxy compound represented by the formula Q-OH (where Q represents organic group which may have a substituent) with 1,4-cyclohexanedicarboxylic acid dichloride or 1,4-cyclohexanedicarboxylic acid; a step (B2) of mixing the reaction solution with a basic compound and water to afford a precipitate; and a step (B3) of mixing the precipitate obtained in step (B2), either in solid or dissolved state, with an acidic aqueous solution. 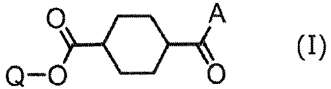 where A represents hydroxyl group or chlorine atom and Q is as defined above.

Through these steps, it is possible to obtain a mixture containing an intermediate compound of interest represented by the formula (I) where A is hydroxyl group.

As with production method A, Q in the general formula (I) preferably represents the organic group represented by the formula (II).

### -Step (B1)-

Step (B1) is the same as step (A1). Thus, in step (B1), an esterification reaction can be effected using the organic solvent, hydroxy compound, 1,4-cyclohexanedicarboxylic acid and 1,4-cyclohexanedicarboxylic acid dichloride described above in step (A1) and optionally using the same base, condensing agent and other additives as those described above. The conditions for the esterification reaction can also be the same as those described above in step (A1). By step (B1), a reaction product is obtained which contains a monoester compound having the specific structure represented by the formula (I) and residual unreacted 1,4-cyclohexanedicarboxylic acid dichloride and/or 1,4-cyclohexanedicarboxylic acid.

### -Step (B2)-

In step (B2), a basic compound and water are mixed with the reaction solution obtained in step (B1) to afford a precipitate. This step ionizes the cyclohexanedicarboxylic acid and 1,4-cyclohexanedicarboxylic acid dichloride (unreacted matter contained in the reaction solution) so that they dissolve in the aqueous phase while allowing a monoester compound represented by the following formula (VI) to precipitate. 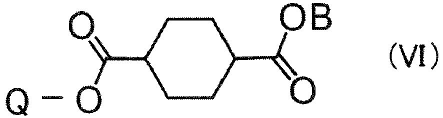 where Q represents the same organic group as defined in the formula (II); and B is a residue of a basic compound described later which is added in step (B2) and represents alkali metal atom or alkaline earth metal atom.

### [Basic Compound]

Any basic compound can be used as long as it is a compound capable of undergoing an acid-base reaction with unreacted cyclohexanedicarboxylic acid and/or 1,4-cyclohexanedicarboxylic acid dichloride. Examples of basic compounds include basic compounds having alkali or alkaline earth metal, such as alkali metal hydrides, alkaline earth metal hydrides, alkali metal hydroxides, alkaline earth metal hydroxides, alkali metal alkyloxylates, and alkaline earth metal alkyloxylates. Preferred are basic inorganic compounds having alkali or alkaline earth metal atom, such as alkali metal hydrides, alkaline earth metal hydrides, alkali metal hydroxides, and alkaline earth metal hydroxides. Specific examples include lithium hydride, sodium hydride, potassium hydride, calcium hydride, lithium hydroxide, sodium hydroxide, potassium hydroxide, calcium hydroxide, sodium t-butoxide, and potassium t-butoxide, with sodium hydroxide being preferred.

### [Water]

Any type of water can be mixed in step (B2), e.g., distilled water or ion-exchanged water can be used.

### [Mixing]

When mixing the basic compound and water with the reaction solution obtained in step (B1), the basic compound can be previously dissolved in water to prepare a basic aqueous solution and the basic aqueous solution can then be added to the reaction solution.

The amount of the basic compound used in step (B2) can be 0.1 moles or more and 3 moles or less per mole of the cyclohexanedicarboxylic acid and/or 1,4-cyclohexanedicarboxylic acid dichloride used in step (B1).

Any amount of water can be used in step (B2) and 200 parts by mass or more and 5,000 parts by mass or less of water can be used per 100 parts by mass of the hydroxy compound used in step (B1).

The mixing time in step (B2) preferably ranges from 1 minute to 72 hours, more preferably 1 minute to 5 hours, even more preferably 10 minutes to 5 hours.

### [Solid-Liquid Separation and Washing]

After step (B2), a solid-liquid separation step can be optionally carried out for separating the reaction product from the solution. Any solid-liquid separation method can be used and examples include filtration and decantation. Further, the resultant solids can be washed and dried by common methods similar to those described in the washing operation in step (A1-1).

### -Step (B3)-

In step (B3), the precipitate obtained in step (B2) is contacted with an acidic aqueous solution. Upon contacting, the precipitate may be directly dispersed in an acidic aqueous solution or may be dissolved into solvent to prepare a solution which is then mixed with the acidic aqueous solution. By this step, the residue of the basic compound represented by symbol B in the compound represented by the formula (VI) produced in step (B2) leaves in the acidic aqueous solution, thereby resulting in an intermediate compound represented by the following formula (VII): 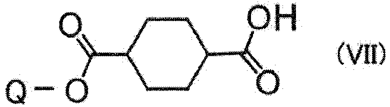 where Q represents the same organic group as defined in the formula (II).

### [Acidic Aqueous Solution]

Any acidic aqueous solution can be used, e.g., common acidic aqueous solutions and acidic buffer solutions can be used. Examples of common acidic aqueous solutions include mineral acids such as aqueous hydrochloric acid solution; and solutions obtained by dissolving organic acids into water, such as aqueous citric acid solution.

The acidic aqueous solution may be an acidic aqueous solution having an acid dissociation constant pKa of 6.5 or less, preferably 6.0 or less, more preferably 5.0 or less. The acid dissociation constant pKa of the acidic aqueous solution means an acid dissociation constant pKa in water at 25°C; in the case of an acid having a plurality of dissociative groups, it means a first dissociation constant. For example, hydrochloric acid has a pKa in water at 25°C of -3.7 and citric acid has a pKa in water at 25°C of 2.9 ("Handbook of Chemistry; Pure Chemistry II, revised 4th ed." edited by The Chemical Society of Japan, published by Maruzen Publishing Co., Ltd., September 30, 1993, II-318, p. 322).

The acidic buffer solution can be an acidic buffer solution having a pH of 5.0 or more and 6.0 or less. Preferably, those exemplified in the description of production method A above can be used.

### [Mixing]

In step (B3), the precipitate obtained in step (B2) is mixed with the acidic aqueous solution in any state, e.g., in dissolved state by dissolving the precipitate into organic solvent or in solid state without dissolving the precipitate into organic solvent. By this step, residual unreacted 1,4-cyclohexanedicarboxylic acid dichloride and/or 1,4-cyclohexanedicarboxylic acid can be removed by being dissolved in an aqueous phase.

As the organic solvent for obtaining the precipitate in dissolved state, any organic solvent can be used and examples include common organic solvents and hydrophilic organic solvents described above. The organic solvent used in this step and the organic solvent used for the esterification reaction in step (B1) may be the same or different.

### [Solid-Liquid Separation and Washing]

After step (B3), a solid-liquid separation step can be optionally carried out for separating the reaction product from the solution. Any solid-liquid separation method can be used and examples include filtration and decantation. Further, the resultant solids can be washed and dried by common methods similar to those described in the washing operation in step (A1-1) to afford a mixture containing an intermediate compound of interest.

### (Compound)

The compound of the present disclosure represented by the following formula (III) is useful as a precursor of a high-quality intermediate compound.

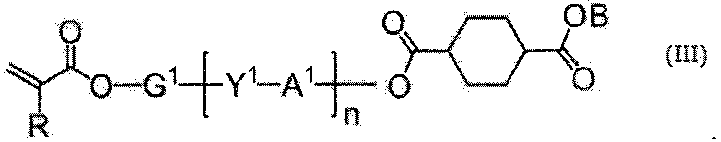

In the formula (III), R represents hydrogen atom or methyl group.

Y¹ represents chemical single bond, -O-, -S-, -O-C(=O)-, -C(=O)-O-, -O-C(=O)-O-, -NR¹-C(=O)-, or -C(=O)-NR¹-. R¹ represents hydrogen atom or C1-C6 alkyl group.

G¹ represents C1-C20 divalent chain aliphatic group which may have a substituent. The chain aliphatic group may include -O-, -S-, -O-C(=O)-, -C(=O)-O-, -O-C(=O)-O-, -NR²-C(=O)-, -C(=O)-NR²-, -NR²-, or -C(=O)- as a spacer, with the proviso that two or more of -O- or two or more of -S- are not present adjacent to each other. R² represents hydrogen atom or C1-C6 alkyl group.

Specific examples of the divalent chain aliphatic group include alkyl group and alkenyl group.

Examples of the alkyl group include C1-C20 alkyl group and specific examples include methyl group, ethyl group, n-propyl group, isopropyl group, n-butyl group, isobutyl group, 1-methylpentyl group, 1-ethylpentyl group, sec-butyl group, t-butyl group, n-pentyl group, isopentyl group, neopentyl group, n-hexyl group, isohexyl group, n-heptyl group, n-octyl group, n-nonyl group, n-decyl group, n-undecyl group, n-dodecyl group, n-tridecyl group, n-tetradecyl group, n-pentadecyl group, n-hexadecyl group, n-heptadecyl group, n-octadecyl group, n-nonadecyl group, and n-icosyl group. C1-20 alkyl group which may have a substituent preferably has 1 to 12 carbon atoms, more preferably 4 to 10 carbon atoms.

Examples of the alkenyl group include C2-C20 alkenyl group and specific examples include vinyl group, propenyl group, isopropenyl group, butenyl group, isobutenyl group, pentenyl group, hexenyl group, heptenyl group, octenyl group, decenyl group, undecenyl group, dodecenyl group, tridecenyl group, tetradecenyl group, pentadecenyl group, hexadecenyl group, heptadecenyl group, octadecenyl group, nonadecenyl group, and icosenyl group. C2-20 alkenyl group which may have a substituent preferably has 2 to 12 carbon atoms.

Examples of the alkynyl group include C2-C20 alkynyl group and specific examples include ethynyl group, propynyl group, 2-propynyl group (propargyl group), butynyl group, 2-butynyl group, 3-butynyl group, pentynyl group, 2-pentynyl group, hexynyl group, 5-hexynyl group, heptynyl group, octynyl group, 2-octynyl group, nonanyl group, decanyl group, and 7-decanyl group.

G¹ is preferably an alkyl group, more preferably unsubstituted n-hexyl group.

When G¹ is a substituted alkyl, alkenyl or alkynyl group, preferred substituents include halogen atoms such as fluorine atom and chlorine atom; cyano group; C1-C20 alkoxy group such as methoxy group, ethoxy group, isopropoxy group and butoxy group; C1-C12 alkoxy group substituted with C1-C12 alkoxy group, such as methoxymethoxy group and methoxyethoxy group; and C1-C12 fluoroalkyl group at least one hydrogen atom of which is substituted with fluorine atom, such as trifluoromethyl group, pentafluoroethyl group, and -CH₂CF₃. Preferred substituents on the alkyl, alkenyl and alkynyl groups are halogen atoms such as fluorine atom and chlorine atom; cyano group; C1-C20 alkoxy group such as methoxy group, ethoxy group, isopropoxy group and butoxy group; and C1-C12 fluoroalkyl group at least one hydrogen atom of which is substituted with fluorine atom, such as trifluoromethyl group, pentafluoroethyl group, and -CH₂CF₃.

The C1-C20 alkyl, C2-20 alkenyl or C2-20 alkynyl group for G¹ may have a plurality of substituents selected from those described above. When G¹ has a plurality of sustituents, each substituent may be the same or different.

A¹ represents C3-C12 divalent aromatic hydrocarbon ring which may have a substituent or C3-C12 divalent cycloaliphatic group which may have a substituent.

The divalent aromatic hydrocarbon ring may be a substituted or unsubstituted divalent aromatic hydrocarbon ring group. The divalent aromatic hydrocarbon ring group is a C3-C12 divalent aromatic group having an aromatic ring structure formed of a hydrocarbon. Specific examples of the divalent aromatic hydrocarbon ring group include 1,4-phenylene group, 1,4-naphthylene group, 1,5-naphthylene group, 2,6-naphthylene group, and 4,4'-biphenylene group.

The divalent cycloaliphatic group may be a substituted or unsubstituted C3-C12 divalent cycloaliphatic group.

Examples of the divalent cycloaliphatic group include cycloalkanediyl group such as cyclopentane-1,3-diyl, cyclohexane-1,4-diyl, 1,4-cycloheptane-1,4-diyl, and cyclooctane-1,5-diyl; and bicycloalkanediyl group such as decahydronaphthalene-1,5-diyl and decahydronaphthalene-2,6-diyl.

A¹ is preferably unsubstituted 1,4-phenylene group.

Examples of substituents on the divalent aromatic hydrocarbon ring group and the divalent cycloaliphatic group include halogen atoms such as fluorine atom, chlorine atom and bromine atom; C1-C6 alkyl group such as methyl group and ethyl group; C1-C5 alkoxy group such as methoxy group and isopropoxy group; nitro group; and cyano group. The aromatic hydrocarbon ring group and cycloaliphatic group may have a plurality of substituents selected from those described above. When they have a plurality of sustituents, each substituent may be the same or different.

B represents alkali metal atom or alkaline earth metal atom. Preferred alkali metal atoms are lithium atom, sodium atom and potassium atom, and a preferred alkaline earth metal atom is calcium atom. Sodium atom is particularly preferred. When B is sodium atom, the precipitating property and therefore the yield of the compound to be a precursor of the intermediate compound represented by the formula (III) improves, so that the production efficiency of the intermediate compound improves. Further, when B is sodium ion, in the solid-liquid separation and washing step, the filtration property and liquid cut-off of the washing solution are good and thereby the production efficiency of the intermediate compound can be further enhanced.

In the formula (III), n represents 0 or 1. In particular, n is preferably 1.

Thus, the compound represented by the formula (III) is preferably a compound represented by the following formula (VIII). Further, in the formula (VIII), B is preferably sodium atom.

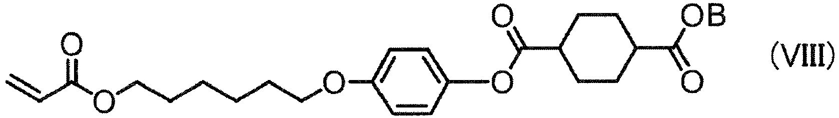

### [Production Method]

The compound of the present disclosure which may be represented by the formula (III) or (VIII) is produced by any method, e.g., in step (B2) of the production method B described above. Compounds and solvents used for the production of the compound of the present disclosure which may be represented by the formula (III) or (VIII) can be those described above with regard to production method B. Also, production conditions can be those described above with regard to production method B.

### (Mixture)

The mixture of the present disclosure is a mixture which contains the compound of the present disclosure represented by the formula (III) described above and a compound represented by the following formula (IV): 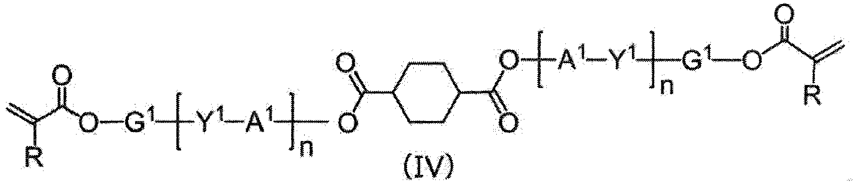 where R, Y¹, G¹ and A¹ each represent the same group or bond as defined in the formula (III). Also, n has the same value as defined in the formula (III). Further, specific examples and preferred examples of these groups and bonds are the same as those described with regard to the formula (III). Thus, the compound represented by the formula (IV) is preferably a compound represented by the following formula (IX):

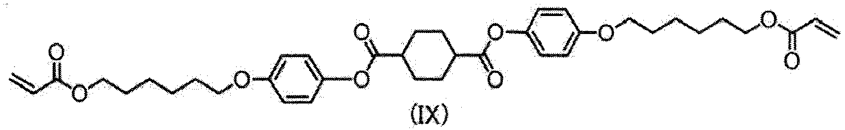

The mixture of the present disclosure contains the compound (IV) in an amount of 50 mol% or less based on the total amount of the compound (III) and the compound (IV). Further, the mixture of the present disclosure contains 1,4-cyclohexanedicarboxylic acid and/or 1,4-cyclohexanedicarboxylic acid dichloride in an amount of less than 5 mol% of the total mixture.

The compound represented by the formula (IV) contained in the mixture of the present disclosure is produced by any method, e.g., by the esterification reaction in step (A1) of production method A or in step (B1) of production method B described above. The mixture of the present disclosure contains the compound (IV) in an amount of 50 mol% or less based on the total amount of the compound (III) and the compound (IV) and is therefore of high quality and can be suitably used as a source of an intermediate compound required for the industrial-scale production of a polymerizable compound.

The mixture of the present disclosure preferably contains the compound (IV) in an amount of 40 mol% or less based on the total amount of the compound (III) and the compound (IV), more preferably 30 mol% or less, even more preferably 20 mol% or less.

The mixture of the present disclosure contains residual unreacted 1,4-cyclohexanedicarboxylic acid and/or 1,4-cyclohexanedicarboxylic acid dichloride in an amount of less than 5 mol% of the total mixture and is therefore of high quality and can be suitably used when producing a polymerizable compound which may be used for the industrial-scale manufacture of an optical film or optically anisotropic product.

### EXAMPLES

### The present disclosure will now be specifically described based on Examples, which however shall not be construed as limiting the scope of the present disclosure.

The amounts of various compounds obtained by the production methods according to Examples were measured by the method described below. Based on the measured values, the production efficiencies of the production methods according to Examples and the qualities of intermediate compounds obtained by the production methods were then evaluated by the methods described below.

The pHs of acidic buffer solutions used in Examples were measured using the portable pH meter D-71 (HORIBA, Ltd.).

### <Amounts of Various Compounds in Mixture>

The amounts of intermediate compound (1) and compound (2) in the mixtures obtained in Examples were measured according to the method described below.

### [Amounts of Intermediate Compound (1) and Compound (2)]

The amount of the intermediate compound (1) in the mixture obtained in each Example was measured by high-performance liquid chromatography (HPLC). To obtain a calibration curve, standards containing three different known concentrations of highly pure (>99.5%) intermediate compound (1) were prepared. Based on the results of analyzing certain amounts of the three standards of different concentrations using an autosampler, a calibration curve was prepared which is a plot of concentration versus peak area of HPLC, and quantification was carried out based on the calibration curve. Quantification of the other compound was carried out in the same manner. In this way, quantification was carried out for each compound.

The conditions of high-performance liquid chromatography were as described below.

### Instrument: Agilent LC 1260

### Column: XDB-C8, 4.6 mm diameter × 250 mm length (Agilent 990967-906)

### Column temperature: 40°C

Mobile phase: acetonitrile/0.1wt% trifluoroacetic acid solution = 60/40 (mass ratio). The mass ratio was altered from 60/40 to 95/5 in a gradient manner over 20 minutes from the beginning of the analysis and then the mobile phase composition ratio was held for 5 minutes (analysis was conducted for a total of 25 minutes).

### <Production Efficiency of Production Method>

The yield of intermediate compound (1) was calculated in mol% based on compound (A) used as a raw material, based on the measured values of the amounts of the compounds obtained above. A high yield of intermediate compound (1) means that intermediate compound (1) could be produced at high efficiency.

### <Quality of Intermediate Compound>

The quality of intermediate compound (1) obtained in each Example was evaluated based on the ratio (mol%) of compound (2) to the total amount of intermediate compound (1) and compound (2) and on the amount of cyclohexane dicarboxylic acid in the mixture obtained in the Example. The amount of cyclohexane dicarboxylic acid in the mixture was measured by ¹³C-NMR spectroscopy (500 MHz, dimethylformamide (DMF)-d₇, 23°C). When the amount of cyclohexane dicarboxylic acid was below the detection limit (i.e., <5 mol%) by ¹³C-NMR spectroscopy under the measurement conditions described above, the mixture was evaluated as being "free" of any residue of unreacted matter. When the mixture contained no or small amount of unreacted cyclohexane dicarboxylic acid, it means that intermediate compound (1) of high quality was obtained.

### (Example 1)

Mixture 1 containing intermediate compound (1) and compound (2) was prepared according to the scheme shown below. In the scheme, "NMP" means N-methyl-2-pyrrolidone and "AcOH/AcONa Buffer" means acetic acid-sodium acetate buffer solution.

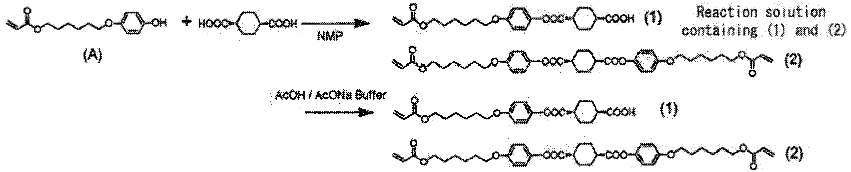

### <Step (A1)>

A three-neck reaction vessel fitted with a thermometer was charged with 41.2 g (239 mmol) of trans-1,4-cyclohexanedicarboxylic acid and 100 g of N-methyl-2-pyrrolidone (NMP) as a hydrophilic organic solvent under nitrogen stream to afford a solution. To the solution were added 12.64 g (47.83 mmol) of 4-(6-acryloyloxy-hex-1-yloxy) phenol (manufactured by DKSH) as a hydroxy compound represented by the formula (A) and 643 mg (5.26 mmol) of 4-(dimethylamino)pyridine as a base. To the solution was slowly added dropwise 11.0 g (57.4 mmol) of 1-(3-dimethylaminopropyl)-3-ethylcarbodiimide hydrochloride as a condensing agent over 1 hour. The total solution was stirred for 15 hours to effect an esterification reaction at room temperature (JIS Z 8703).

### <Step (A2)>

To the reaction solution obtained in step (A1) was slowly added dropwise 500 ml of 1 mol/L acetic acid-sodium acetate buffer solution (pH 5.5) as an acidic buffer solution. The acidic buffer solution was prepared so that the concentration of total acetate ions in the solution was 1 mol/L. After the addition, the solution was stirred for 1 hour to afford a slurry.

### <Filtration Step>

The slurry was filtrated and the filter cake (solid) was washed with a 1:1 (mass ratio) mixed solvent of methanol and water. The washed solid was dried in a vacuum drier to afford 18.10 g of mixture 1 as a white solid.

### <Evaluation>

The white solid (crystal) was analyzed by HPLC in accordance with the method described above for quantification of monoester intermediate compound (1) and diester compound (2) according to the calibration curve. Mixture 1 was found to contain 13.33 g (31.85 mmol) of intermediate compound (1) and 4.77 g (7.18 mmol) of compound (2). The white solid was also analyzed by ¹³C-NMR spectroscopy in accordance with the procedure described above to confirm that mixture 1 was substantially free of cyclohexane dicarboxylic acid. The yield of intermediate compound (1) based on compound (A) as calculated in accordance with the procedure described above was found to be 66.6 mol%.

### (Example 2)

Mixture 2 containing intermediate compound (1) and compound (2) was obtained by a synthesis method wherein trans-1,4-cyclohexanedicarboxylic acid dichloride was used as a raw material instead of trans-1,4-cyclohexanedicarboxylic acid 1 to afford a reaction solution. The reaction scheme was as shown below. In the following scheme, "THF" means tetrahydrofuran.

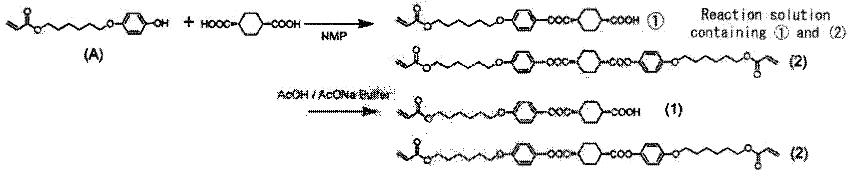

### <Step (A1)>

A three-neck reaction vessel fitted with a thermometer was charged with 10.0 g (47.83 mmol) of trans-1,4-cyclohexanedicarboxylic acid dichloride and 100 ml of tetrahydrofuran (THF) as a hydrophilic organic solvent under nitrogen stream. To the solution was added 12.04 g (45.55 mmol) of 4- (6-acryloyloxy-hex-1-yloxy)phenol (manufactured by DKSH) as a hydroxy compound and the reaction vessel was immersed into ice bath to adjust the inner temperature of the reaction solution to 0°C. 4.83 g (47.83 mmol) of triethylamine as a base was then slowly added dropwise over 5 minutes while maintaining the internal temperature of the reaction solution at 10°C or below. After completion of the addition, the total solution was stirred for a further 1 hour while maintaining the temperature to 10°C or lower.

### <Step (A2)>

As an acidic buffer solution, 150 ml of 1 mol/L acetic acid-sodium acetate buffer solution (pH 5.5) prepared in the same manner as in Example 1 was slowly added dropwise to the reaction solution obtained in step (A1). After completion of the addition, the solution was stirred for 2 hours to afford a slurry.

### <Filtration Step>

The slurry was filtrated and the filter cake (solid) was washed with a 1:1 (mass ratio) mixed solvent of methanol and water. The washed solid was dried in a vacuum drier to afford 17.09 g of mixture 2 as a white solid.

### <Evaluation>

Various evaluations were made on the obtained white solid (crystal) in the same manner as in Example 1. The results are shown in Table 1.

### (Example 3)

Mixture 3 containing intermediate compound (1) and compound (2) was prepared according to the following scheme. In this example, mixture 3 was obtained through step (B1) which is similar to step (A1) in Example 1, steps (B2) and (B3) described in detail below, and first and second filtration/washing steps. In the following scheme, "NaOHaq." means an aqueous sodium hydroxide solution and "HClaq." means an aqueous hydrochloric acid solution.

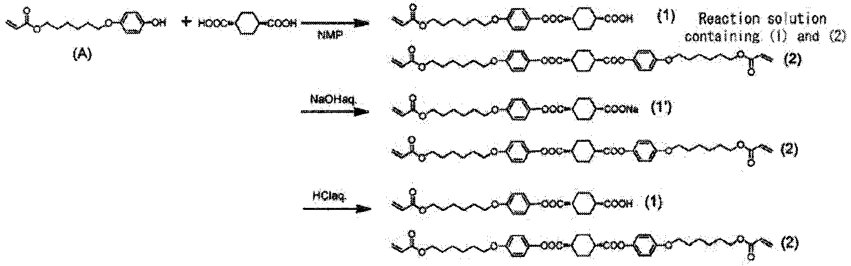

### <Step (B1)>

A three-neck reaction vessel fitted with a thermometer was charged with 41.2 g (239 mmol) of trans-1,4-cyclohexanedicarboxylic acid and 100 g of N-methyl-2-pyrrolidone (NMP) as a hydrophilic organic solvent under nitrogen stream. To the resultant solution were added 12.64 g (47.83 mmol) of 4-(6-acryloyloxy-hex-1-yloxy)phenol (manufactured by DKSH) as a hydroxy compound represented by the formula (A) and 643 mg (5.26 mmol) of 4-(dimethylamino) pyridine as a base. To the solution was slowly added dropwise 11.0 g (57.4 mmol) of 1-(3-dimethylaminopropyl)-3-ethylcarbodiimide hydrochloride as a condensing agent over 1 hour. The total solution was stirred for 15 hours to effect an esterification reaction at room temperature (JIS Z 8703).

### <Step (B2)>

To the reaction solution obtained in step (B1) was slowly added dropwise a separately prepared basic aqueous solution consisting of 2.4 g (60 mmol) of sodium hydroxide as a basic compound and 165 ml of distilled water. The solution was then stirred for 2 hours to afford a precipitate.

### <First Filtration/Washing Step>

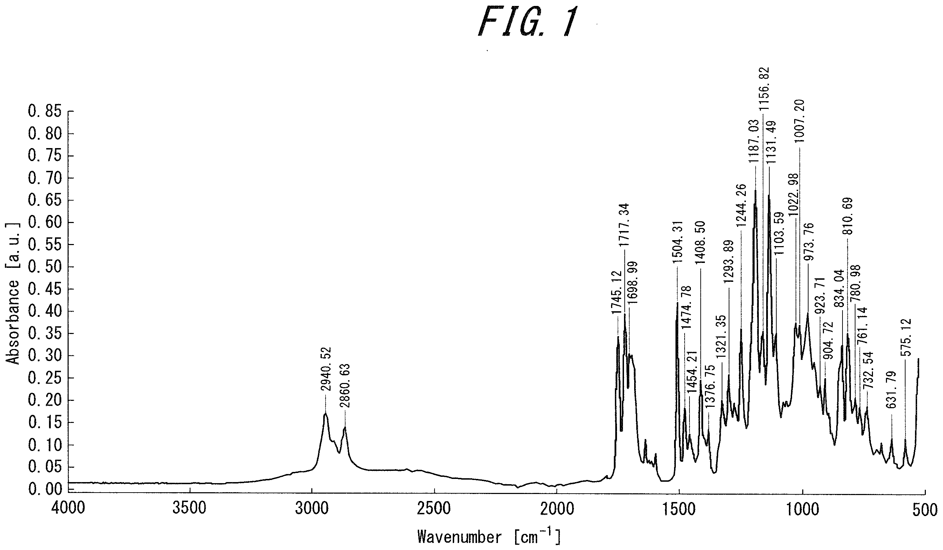

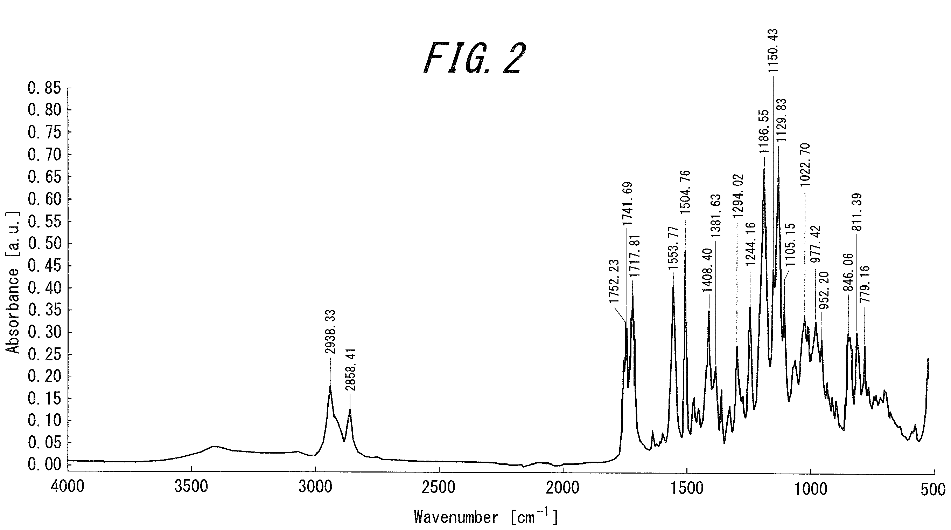

The precipitate obtained in step (B2) was recovered by filtration and washed with water. The resultant solid was dried in a vacuum drier to afford 18.9 g of white solid containing compound (1'). For the obtained white solid, an infrared absorption spectrum was measured by infrared spectroscopy. The result is shown in FIG. 2. Because the carboxylate anion (COO⁻) peak was detected near wavenumber of 1,550 cm⁻¹ (peak at wavenumber of 1,553.77 cm⁻¹ in FIG. 2), it was confirmed that the white solid contained compound (1'). For comparison, an infrared absorption spectrum was measured also for mixture 3 obtained by the production method according to Examples. The result is shown in FIG. 1. As evident from FIG. 1, in the infrared absorption spectrum of mixture 3, no peak was confirmed near wavenumber of 1,550 cm⁻¹, which reveals that mixture 3 did not contain the carboxylate anion (COO⁻).

### <Step (B3)>

18.9 g of the white solid obtained in the first filtration/washing step was re-dissolved in 50 g of tetrahydrofuran as a hydrophilic organic solvent to afford a solution. To this solution was slowly added dropwise 70 g of 1.0 mol/L aqueous hydrochloric acid solution as an acidic aqueous solution. After completion of the addition, the solution was stirred for 1 hour to precipitate a solid. The added aqueous hydrochloric acid solution had a pH of 1 as measured with the portable pH meter D-71 (HORIBA, Ltd.).

### <Second Filtration/Washing Step>

The solid precipitated in step (B3) was filtered off and washed with water. The resultant solid was dried in a vacuum drier to afford 17.96 g of mixture 3 as a white solid.

### <Evaluation>

Various evaluations were made on the obtained white solid (crystal) in the same manner as in Example 1. The results are shown in Table 1.

### (Example 4)

Mixture 4 containing intermediate compound (1) and compound (2) was prepared according to the following scheme. In this example, an operation similar to that in Example 3 was carried out except that as the acidic aqueous solution used in step (B3), 100 ml of 1 mol/L acetic acid-sodium acetate buffer solution (pH 5.5) prepared in the same manner as in Example 1 was used instead of 70 g of aqueous hydrochloric acid solution.

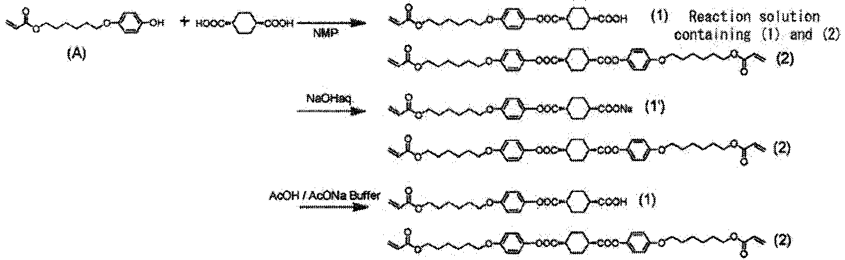

### <Second Filtration/Washing Step>

In the second filtration/washing step, an operation similar to that in Example 3 was carried out to afford 18.04 g of mixture 4 as a white solid.

### <Evaluation>

Various evaluations were made on the obtained white solid (crystal) in the same manner as in Example 1. The results are shown in Table 1.

### (Example 5)

In this example, mixture 5 was produced as in Example 3 mainly except that step (B1) was changed to a synthesis method wherein trans-1,4-cyclohexanedicarboxylic acid dichloride is used, the amounts of the basic compound and distilled water used in step (B2) were reduced, and the amount of the acidic aqueous solution used in step (B3) was reduced. The reaction scheme was as shown below.

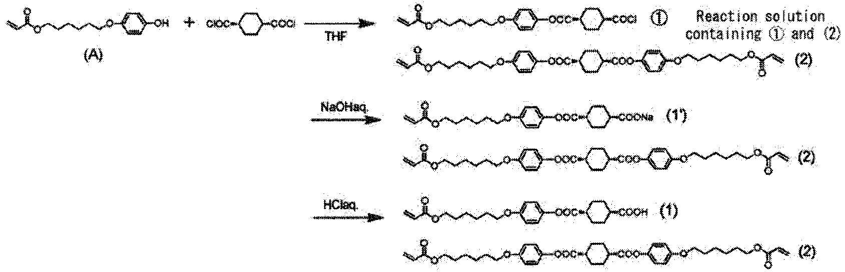

### <Step (B1)>

A three-neck reaction vessel fitted with a thermometer was charged with 10.0 g (47.83 mmol) of trans-1,4-cyclohexanedicarboxylic acid dichloride and 50 ml of tetrahydrofuran (THF) under nitrogen stream. To the solution was added 12.04 g (45.55 mmol) of 4-(6-acryloyloxy-hex-1-yloxy)phenol (manufactured by DKSH) as a hydroxy compound and the reaction vessel was immersed into ice bath to adjust the inner temperature of the reaction solution to 0°C. 4.83 g (47.83 mmol) of triethylamine as a base was then slowly added dropwise over 5 minutes while maintaining the internal temperature of the reaction solution at 10°C or below. After completion of the addition, the total solution was stirred for a further 1 hour while maintaining the temperature to 10°C or lower.

### <Step (B2)>

To the reaction solution obtained in step (B1) was slowly added dropwise a separately prepared basic aqueous solution consisting of 0.48 g (12 mmol) of sodium hydroxide as a basic compound and 40 ml of distilled water. The solution was then stirred for 2 hours and filtrated to recover the precipitate.

### <First Filtration/Washing Step>

The precipitate obtained in step (B2) was washed with a 1:1 (mass ratio) mixed solvent of methanol and water. The washed solid was dried in a vacuum drier to afford 17.85 g of white solid containing compound (1'). For the white solid, infrared spectroscopy was carried out as in Example 3 to confirm that the white solid contained compound (1').

### <Step (B3)>

17.85 g of the white solid obtained in the first filtration/washing step was re-dissolved in 50 g of tetrahydrofuran as a hydrophilic organic solvent. To this solution was slowly added dropwise 50 g of 0.5 mol/L aqueous hydrochloric acid solution as an acidic aqueous solution. After completion of the addition, the solution was stirred for 1 hour to precipitate a solid.

### <Second Filtration/Washing Step>

The solid precipitated in step (B3) was filtered off and washed with a 1:1 (mass ratio) mixed solvent of methanol and water. The resultant solid was dried in a vacuum drier to afford 17.07 g of mixture 5 as a white solid.

### <Evaluation>

Various evaluations were made on the obtained white solid (crystal) in the same manner as in Example 1. The results are shown in Table 1.

### (Example 6)

In this example, an operation similar to that in Example 5 was carried out to produce mixture 6 except that as the acidic aqueous solution used in step (B3), 1 mol/L acetic acid-sodium acetate buffer solution was used instead of aqueous hydrochloric acid solution. The following describes a step in which an operation that is different from that in Example 5 was carried out and a step in which an operation that produced a different amount of product was carried out. The reaction scheme was as shown below.

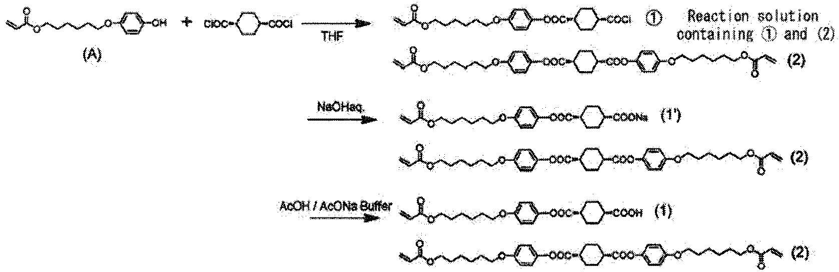

### <Step (B3)>

17.85 g of the white solid obtained in the first filtration/washing step similar to that in Example 5 was re-dissolved in 50 g of tetrahydrofuran. To this solution was slowly added dropwise 100 ml of acetic acid-sodium acetate buffer solution (pH 5.5) prepared in the same manner as in Example 1. After completion of the addition, stirring was carried out for 1 hour to precipitate a solid.

### <Second Filtration/Washing Step>

The solid precipitated in step (B3) was filtered off and washed with a 1:1 (mass ratio) mixed solvent of methanol and water. The resultant solid was dried in a vacuum drier to afford 17.04 g of mixture 6 as a white solid.

### <Evaluation>

Various evaluations were made on the obtained white solid (crystal) in the same manner as in Example 1. The results are shown in Table 1.

### (Example 7)

In this example, an operation similar to that in Example 3 was carried out to produce mixture 7 except that the amounts of sodium hydroxide (basic compound) and distilled water added in step (B2) were changed. The following describes a step in which an operation that is different from that in Example 3 was carried out and a step in which an operation that produced a different amount of product was carried out. The reaction scheme was as shown below.

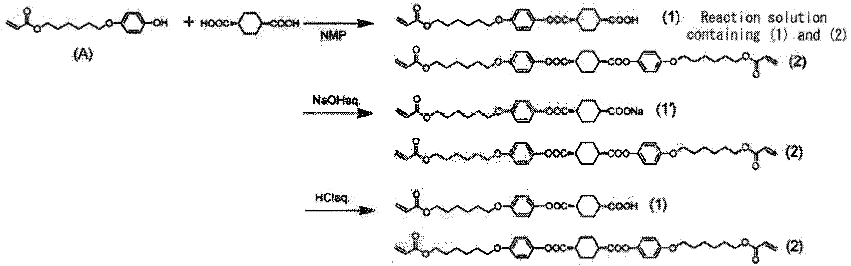

### <Step (B2)>

To the reaction solution obtained in step (B1) similar to that in Example 3 was slowly added dropwise a separately prepared basic aqueous solution consisting of 18 g (450 mmol) of sodium hydroxide as a basic compound and 500 ml of distilled water. The solution was then stirred for 2 hours and filtrated to recover the precipitate.

### <First Filtration/Washing Step>

The precipitate obtained in step (B2) was washed with a 1:1 (mass ratio) mixed solvent of methanol and water. The washed solid was dried in a vacuum drier to afford 18.9 g of white solid containing compound (1'). For the white solid, infrared spectroscopy was carried out as in Example 3 to confirm that the white solid contained compound (1').

### <Step (B3)>

18.9 g of the white solid obtained in the first filtration/washing step was re-dissolved in 50 g of tetrahydrofuran as a hydrophilic organic solvent to afford a solution. To this solution was slowly added dropwise 70 g of 1.0 mol/L aqueous hydrochloric acid solution. After completion of the addition, stirring was carried out for 1 hour to precipitate a solid.

### <Second Filtration/Washing Step>

The solid precipitated in step (B3) was filtered off and washed with a 1:1 (mass ratio) mixed solvent of methanol and water. The resultant solid was dried in a vacuum drier to afford 17.95 g of mixture 7 as a white solid.

### <Evaluation>

Various evaluations were made on the obtained white solid (crystal) in the same manner as in Example 1. The results are shown in Table 1.

### (Example 8)

In this example, an operation similar to that in Example 7 was carried out to produce mixture 8 except that as the acidic aqueous solution used in step (B3), 1 mol/L acetic acid-sodium acetate buffer solution was used instead of aqueous hydrochloric acid solution. The following describes a step in which an operation that is different from that in Example 7 was carried out. The reaction scheme was as shown below.

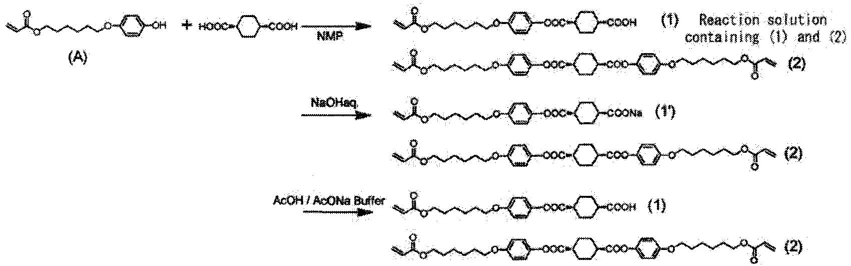

### <Step (B3)>

18.9 g of the solid obtained in the first filtration/washing step similar to that in Example 7 was re-dissolved in 50 g of tetrahydrofuran. To this solution was slowly added dropwise 100 ml of acetic acid-sodium acetate buffer solution (pH 5.5) prepared in the same manner as in Example 1. After completion of the addition, stirring was carried out for 1 hour to precipitate a solid.

### <Second Filtration/ Washing Step to Evaluation>

An operation similar to that in Example 7 was carried out for the solid precipitated in step (B3) to afford 18.11 g of mixture 8. Various evaluations were made on mixture 8 in the same manner as in Example 1. The results are shown in Table 1.

### (Example 9)

In this example, an operation similar to that in Example 5 was carried out to produce mixture 9 except that the amounts of the basic compound and distilled water added in step (B2) were changed as shown in Table 1. The reaction scheme was as shown below, which is the same as that in Example 5.

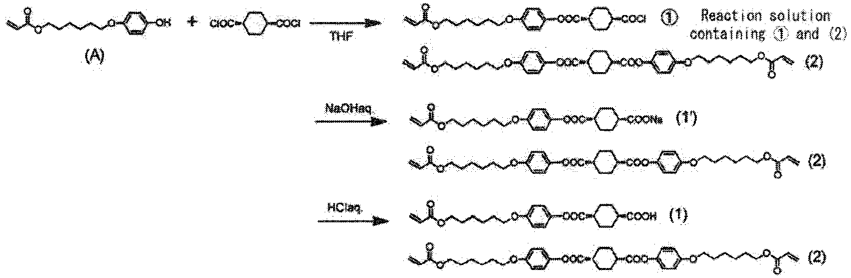

### <Step (B2)>

To the reaction solution obtained in step (B1) similar to that in Example 5 was slowly added dropwise a separately prepared basic aqueous solution consisting of 2.4 g (60 mmol) of sodium hydroxide as a basic compound and 50 ml of distilled water. The solution was then stirred for 2 hours and filtrated to recover the precipitate.

### <First Filtration/Washing Step>

The precipitate obtained in step (B2) was washed with a 1:1 (mass ratio) mixed solvent of methanol and water. The washed solid was dried in a vacuum drier to afford 18.1 g of white solid containing compound (1'). For the white solid, infrared spectroscopy was carried out as in Example 3 to confirm that the white solid contained compound (1').

### <Step (B3) to Evaluation>

An operation similar to that in Example 5 was carried out for 18.1 g of the white solid obtained in the first filtration/washing step to afford 17.16 g of mixture 9. Various evaluations were made on mixture 9 in the same manner as in Example 1. The results are shown in Table 1.

### (Example 10)

In this example, an operation similar to that in Example 9 was carried out to produce 17.19 g of mixture 10 except that as the acidic aqueous solution used in step (B3), 100 ml of 1 mol/L acetic acid-sodium acetate buffer solution (pH 5.5) was used instead of aqueous hydrochloric acid solution. Various evaluations were made on mixture 10 in the same manner as in Example 1. The results are shown in Table 1. The reaction scheme was as shown below.

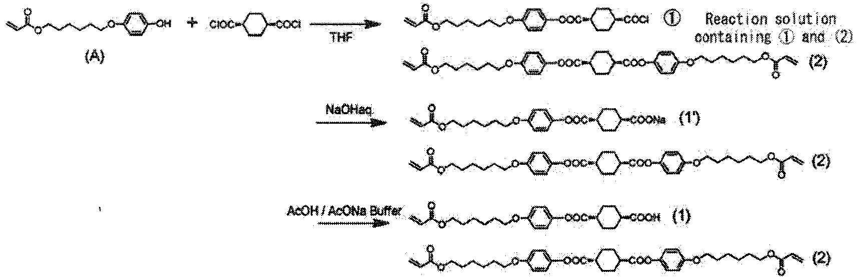

### (Example 11)

In this example, step (A1) was carried out as in Example 1, followed by the following step (A1-1) to produce mixture 11. The reaction scheme was as shown below.

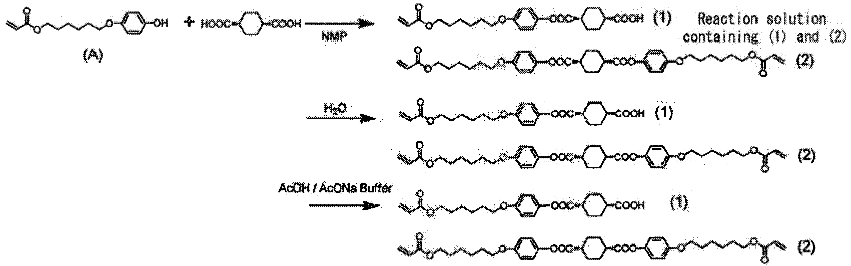

### <Step (A1-1)>

To the solution obtained in step (A1) similar to that in Example 1 was slowly added dropwise 500 ml of distilled water. The solution was stirred for 30 minutes and filtrated, and the filter cake was washed with a 1:1 (mass ratio) mixed solvent of methanol and water. The washed solid was dried in a vacuum drier to afford 18.43 g of white solid containing intermediate compound (1).

### <Step (A2)>

18.43 g of the white solid obtained in step (Al-1) was re-dissolved in 50 g of tetrahydrofuran. To this solution was slowly added dropwise 500 ml of 1mol/L acetic acid-sodium acetate buffer solution (pH 5.5) prepared in the same manner as in Example 1. After completion of the addition, stirring was carried out for 1 hour to precipitate a solid.

### <Filtration Step>

The precipitated solid was filtered off and washed with a 1:1 (mass ratio) mixed solvent of methanol and water. The washed solid was dried in a vacuum drier to afford 18.03 g of mixture 11 as a white solid.

### <Evaluation>

Various evaluations were made on the obtained white solid (crystal) in the same manner as in Example 1. The results are shown in Table 1.

### (Example 12)

In this example, as described below, mixture 12 was produced as in Example 11 mainly except that step (A1) was changed to a synthesis method wherein trans-1,4-cyclohexanedicarboxylic acid dichloride is used, the amount distilled water added in step (A1-1) was changed to 50 ml, and the amount of the acidic aqueous solution used in step (A2) was reduced. Various evaluations were made on mixture 12 in the same manner as in Example 1. The results are shown in Table 1. The reaction scheme was as shown below.

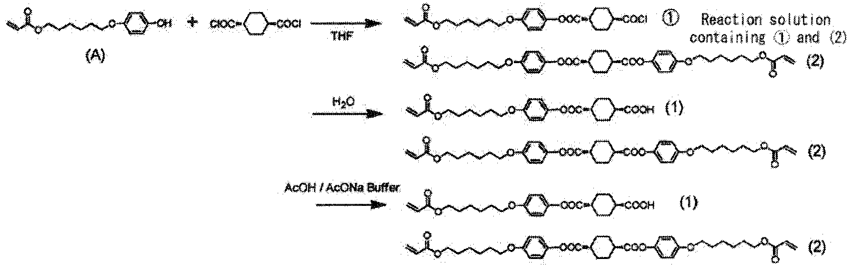

### <Step (A1)>

A three-neck reaction vessel fitted with a thermometer was charged with 10.0 g (47.83 mmol) of trans-1,4-cyclohexanedicarboxylic acid dichloride and 50 ml of tetrahydrofuran (THF) as a hydrophilic organic solvent under nitrogen stream. To the solution was added 12.04 g (45.55 mmol) of 4-(6-acryloyloxy-hex-1-yloxy)phenol (manufactured by DKSH) as a hydroxy compound and the reaction vessel was immersed into ice bath to adjust the inner temperature of the reaction solution to 0°C. 4.83 g (47.83 mmol) of triethylamine as a base was then slowly added dropwise over 5 minutes while maintaining the internal temperature of the reaction solution at 10°C or below. After completion of the addition, the total solution was stirred for a further 1 hour while maintaining the temperature to 10°C or lower.

### <Step (A1-1)>

To the reaction solution obtained in step (A1) was slowly added dropwise 50 ml of distilled water. The solution was then stirred for 30 minutes and filtrated, and the filter cake was washed with a 1:1 (mass ratio) mixed solvent of methanol and water. The washed solid was dried in a vacuum drier to afford 17.50 g of white solid containing intermediate compound (1).

### <Step (A2)>

17.50 g of the white solid obtained in step (A1-1) was re-dissolved in 50 g of tetrahydrofuran. To this solution was slowly added dropwise 100 ml of acetic acid-sodium acetate buffer solution (pH 5.5) prepared in the same manner as in Example 1. After completion of the addition, the solution was stirred for 1 hour to precipitate a solid.

### <Filtration Step>

The precipitated solid was filtered off and washed with a 1:1 (mass ratio) mixed solvent of methanol and water. The washed solid was dried in a vacuum drier to afford 17.16 g of mixture 12 as a white solid.

In Table 1, "CYDA" means trans-1,4-cyclohexanedicarboxylic acid, "CYDA-C1" means trans-1,4-cyclohexanedicarboxylic acid dichloride, "NMP" means N-methyl-2-pyrrolidone, "THF" means tetrahydrofuran, and "AcOH/AcONa" means acetic acid-sodium acetate aqueous solution,

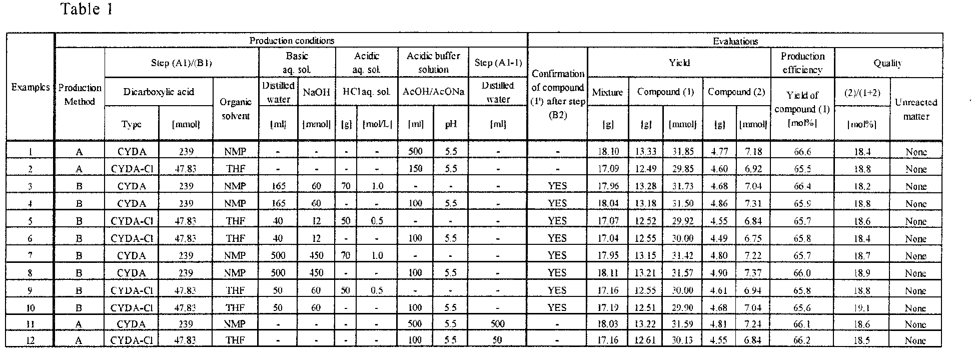

The method of the present disclosure which includes: esterifying a hydroxy compound having a specific structure represented by the general formula (A) in the scheme shown above with a specific dicarboxylic acid compound to afford a reaction product containing a monoester compound having a specific structure; and mixing the resultant reaction product at least with an acidic buffer solution or a basic compound as described in detail in Examples 1 to 12 provided an intermediate compound of high quality, with the resultant mixture containing small amounts of diester compound and unreacted matter. The production efficiency of the production methods described in detail in Examples 1 to 12 was superior as the operability of each step was good. In addition, the productions methods described in detail in Examples 1 to 12 had high production efficiency, with an intermediate compound being obtained at high yield.

In particular, the production methods according to Examples 3 to 10 showed superior filtration property particularly in the second filtration/washing step, producing a mixture containing intermediate compound (1) at high efficiency.

The production methods according to Examples 3 to 10 produced a high-quality intermediate compound with compound (1') being produced during the process.

### INDUSTRIAL APPLICABILITY

According to the production method of the present disclosure, it is possible to produce a high-quality intermediate compound at high yield. According to the compound of present disclosure or a mixture containing the compound, it is also possible to produce a high-quality intermediate compound at high yield.

## Claims

1. A method of producing a compound, comprising:
a step (A1) of obtaining a reaction product which contains a monoester compound represented by the following formula (I) and 1,4-cyclohexanedicarboxylic acid dichloride and/or 1,4-cyclohexanedicarboxylic acid, by reacting in an organic solvent a hydroxy compound represented by the formula Q-OH (where Q represents organic group which may have a substituent) with 1,4-cyclohexanedicarboxylic acid dichloride and/or 1,4-cyclohexanedicarboxylic acid; and
a step (A2) of mixing the reaction product with an acidic buffer solution, 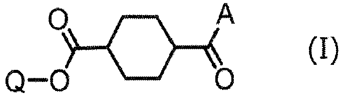 where A represents hydroxyl group or chlorine atom and Q is as defined above.

2. The method according to claim 1, wherein the organic solvent is a hydrophilic organic solvent.

3. The method according to claim 1 or 2, further comprising a step (A1-1) of mixing the reaction product with water between the step (A1) and the step (A2).

4. The method according to any one of claims 1 to 3, wherein the acidic buffer solution has a pH of 5.0 or more and 6.0 or less.

5. A method of producing a compound, comprising:
a step (B1) of obtaining a reaction solution which contains a monoester compound represented by the following formula (I) and 1,4-cyclohexanedicarboxylic acid dichloride and/or 1,4-cyclohexanedicarboxylic acid, by reacting in an organic solvent a hydroxy compound having the formula Q-OH (where Q represents organic group which may have a substituent) with 1,4-cyclohexanedicarboxylic acid dichloride or 1,4-cyclohexanedicarboxylic acid;
a step (B2) of mixing the reaction solution with a basic compound and water to afford a precipitate; and
a step (B3) of mixing the precipitate obtained in step (B2), either in solid or dissolved state, with an acidic aqueous solution, 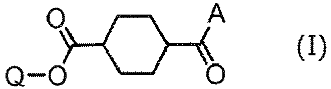 where A represents hydroxyl group or chlorine atom and Q is as defined above.

6. The method according to claim 5, wherein the acidic aqueous solution has an acid dissociation constant pKa of 6.5 or less.

7. The method according to claim 5, wherein the acidic aqueous solution is an acidic buffer solution having a pH of 5.0 or more and 6.0 or less.

8. The method according to any one of claims 1 to 4 and 7, wherein the acidic buffer solution is a mixed solution of acetic acid and sodium acetate, or a mixed solution of potassium hydrogen phthalate and sodium hydroxide.

9. The method according to any one of claims 1 to 8, wherein the organic group Q of the compound represented by the formula (I) is represented by the following formula (II): 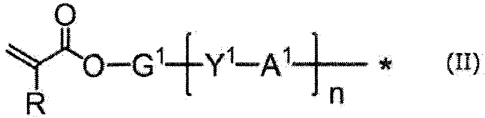 where R represents hydrogen or methyl group; Y¹ represents chemical single bond, -O-, -S-, -O-C(=O)-, -C(=O)-O-, -O-C(=O)-O-, -NR¹-C(=O)-, or -C(=O)-NR¹- (where R¹ represents hydrogen atom or C1-C6 alkyl group); G¹ represents C1-C20 divalent chain aliphatic group which may have a substituent (where the chain aliphatic group may include -O-, -S-, -O-C(=O)-, -C(=O)-O-, -O-C(=O)-O-, -NR²-C(=O)-, -C(=O)-NR²-, -NR²-, or -C(=O)- (where R² represents hydrogen atom or C1-C6 alkyl group) as a spacer, with the proviso that two or more of -O- or two or more of -S- are not present adjacent to each other); A¹ represents C3-C12 divalent aromatic hydrocarbon ring which may have a substituent or C3-C12 divalent cycloaliphatic group which may have a substituent; and n represents 0 or 1.

10. A compound represented by the following formula (III): 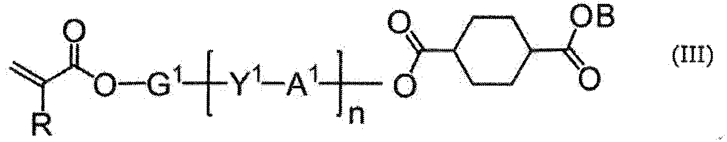 where R represents hydrogen or methyl group; Y¹ represents chemical single bond, -O-, -S-, -O-C(=O)-, -C(=O)-O-, -O-C(=O)-O-, -NR¹-C(=O)-, or -C(=O)-NR¹- (where R¹ represents hydrogen atom or C1-C6 alkyl group); G¹ represents C1-C20 divalent chain aliphatic group which may have a substituent (where the chain aliphatic group may include -O-, -S-, -O-C(=O)-, -C(=O)-O-, -O-C(=O)-O-, -NR²-C(=O)-, -C(=O)-NR²-, -NR²-, or -C(=O)- (where R² represents hydrogen atom or C1-C6 alkyl group) as a spacer, with the proviso that two or more of -O- or two or more of -S- are not present adjacent to each other); A¹ represents C3-C12 divalent aromatic hydrocarbon ring which may have a substituent or C3-C12 divalent cycloaliphatic group which may have a substituent; B represents alkali metal atom or alkaline earth metal atom; and n represents 0 or 1.

11. A mixture comprising:
the compound represented by the formula (III) defined in claim 10; and
a compound represented by the following formula (IV),
wherein the mixture contains the compound (IV) in an amount of 50 mol% or less based on a total amount of the compound (III) and the compound (IV) and contains 1,4-cyclohexanedicarboxylic acid and/or 1,4-cyclohexanedicarboxylic acid dichloride in an amount of less than 5 mol% of the total mixture, 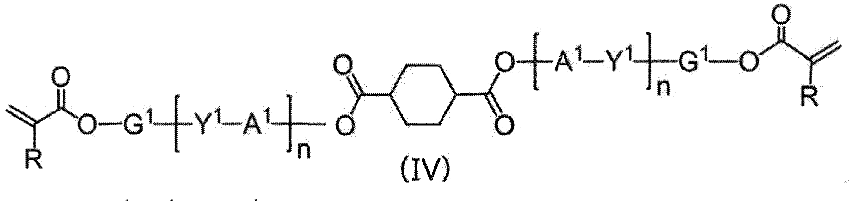 where R, Y¹, G and A¹ each represent the same group or bond as defined in the formula (III); and n has the same value as defined in the formula (III).

12. The compound according to claim 10, wherein in the formula (III), G¹ is unsubstituted hexyl group, Y¹ is -O-, A¹ is unsubstituted 1,4-phenylene group, and n is 1.

13. The mixture according to claim 11, wherein in the formulas (III) and (IV), G¹ is unsubstituted hexyl group, Y¹ is -O-, A¹ is unsubstituted 1,4-phenylene group, and n is 1.
